# EUROPÄISCHE PATENTSCHRIFT

(11) **EP 1 127 618 B1**
(45) Veröffentlichungstag und Bekanntmachung des Hinweises auf die Patenterteilung: **29.03.2006**
(21) Anmeldenummer: 01103199.4
(22) Anmeldetag: 12.02.2001
(51) Int. Cl.: B01J 35/02, C07C 17/156, C07C 51/25, C07C 51/235

(54) **Geformte Katalysatoren**
Shaped catalysts
Catalyseurs mis en forme

(30) Priorität: 25.02.2000 DE 10009017
(43) Veröffentlichungstag der Anmeldung: 29.08.2001
(73) Patentinhaber: BASF Aktiengesellschaft, 67056 Ludwigshafen (DE)
(72) Erfinder: Walsdorff, Christian, Dr., 67061 Ludwigshafen (DE); Hofstadt, Otto, 67346 Speyer (DE); Felder, Raimund, Dr., 67434 Neustadt (DE); Meissner, Ruprecht, 67273 Weisenheim (DE); Harth, Klaus, Dr., 67317 Altleiningen (DE)

(56) Entgegenhaltungen:
- EP-A- 0 017 865
- EP-A- 0 184 790
- EP-A- 0 461 431
- WO-A-99/19065
- GB-A- 893 987

## Beschreibung

Die vorliegende Erfindung betrifft neue geformte Katalysatoren für heterogen katalysierte Reaktionen in Form von Hohlzylindern oder Ringtabletten, deren Stirnflächen sowohl zum Außenrand als auch zum Rand der Innenbohrung hin abgerundet sind, die also keine rechtwinkligen Kanten aufweisen.

Aus der EP-A-184 790 sind bereits geformte Katalysatoren für heterogen katalysierte Reaktionen in Form eines Hohlzylinders bekannt, die am äußeren Zylinderrand sogenannte gekrümmte "Stirnflächen" besitzen.

Aus der WO-A-99/48606 sind Katalysatoren in Form von Ringtabletten mit den Abmessungen 5 mm x 5 mm x 2 mm (Höhe x Durchmesser x Durchmesser des Innenlochs) bekannt.

WO-A-99/19065 (EP-A-1052018) beschreibt geformte Katalysatoren, die im Querschnitt durchgehend gerundete und doughnut-ähnliche Formen ohne plane Bereiche bzw. keine parallel zueinander stehende plane Bereiche aufweisen.

Die geformten Katalysatoren gemäß US-A-4170569 sind annähernd kugelförmige Formkörper, die an keiner Stelle parallele Innen- und Außenwände aufweisen.

Diese Katalysatoren lassen jedoch noch zu wünschen übrig. Insbesondere ist der Druckverlust einer Schüttung dieser Katalysatoren noch nicht optimal.

Der vorliegenden Erfindung lag daher die Aufgabe zugrunde, geformte Katalysatoren für heterogen katalysierte Reaktionen mit verbesserten Eigenschaften, insbesondere mit einem verringerten Druckverlust, bereitzustellen.

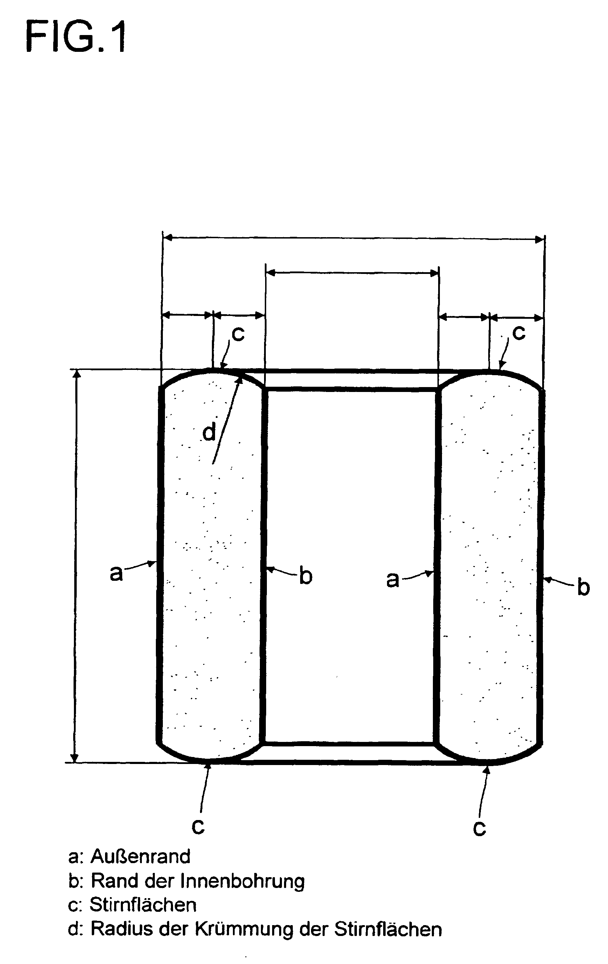

Demgemäß wurden neue und verbesserte geformte Katalysatoren für heterogen katalysierte Reaktionen in Form von Hohlzylindern oder Ringtabletten gefunden, die dadurch gekennzeichnet sind, dass im Querschnitt die äußere Wandfläche (Außenrand) und die innere Wandfläche (Rand der Innenbohrung) weitgehend eben und parallel zueinander angeordnet sind, während die Stirnflächen durchgehend so abgerundet sind, dass - entlang der Innenbohrung betrachtet - sich der Katalysatorkörper sowohl von Außenrand als auch vom Rand der Innenbohrung her verjüngt (Fig. 1).

Die erfindungsgemäßen Katalysatoren werden nach an sich bekannten Verfahren hergestellt, beispielsweise durch Fällung der Oxide, Oxidhydrate, Hydroxide oder anderer schwerlöslicher Verbindungen der Komponenten aus Lösungen ihre Salze, oder durch intensives Vermischen der oxidischen und/oder salzförmigen Ausgangsstoffe, die gegebenenfalls in Wasser oder organischen Lösungsmitteln gelöst oder/und suspendiert sind, anschließende Trocknung und gegebenenfalls thermische Zersetzung der Salze, Vermahlen zu einer geeigneten tablettierfähigen Teilchengröße, Verformung zu der erfindungsgemäßen Form in geeigneten Tablettiermaschinen und gegebenenfalls anschließende thermische Behandlung bei erhöhten Temperaturen in oxidierender, reduzierender oder inerter Atmosphäre. Der Katalysatormasse können in irgendeiner Herstellungsstufe der Verformung Formhilfsmittel wie Graphit, Ruß, Stearinsäure, Stärke, Polyacrylsäure, Mineralöl, pflanzliches Ö1, Methylcellulose u.a., sowie Verstärkungsmittel wie anorganische Fasern, beispielsweise Glasfasern oder dgl., oder anorganische Pulver beispielsweise Metallpulver, Metallflocken, sogenannte inerte Trägerstoffe, beispielsweise SiO₂, Metallsilikate und Aluminiumsilikate, Aluminiumoxide, Aluminiumhydroxide, Aluminiumoxidhydrate, MgO, TiO₂, ZrO₂, Nb₂O₃, Bimsstein, Siliziumcarbid und/oder Magnesiumsilikate zugegeben werden..

Die erfindungsgemäßen geformten Katalysatoren für heterogen katalysierte Reaktionen, sind in der Form Hohlzylinder oder Ringtabletten aus katalytisch aktiven Material oder bevorzugt aus einem zu Hohlzylindern oder Ringtabletten geformten inerten Trägermaterial aufgebaut, auf das eine katalytisch aktive Masse aufgebracht ist, wobei der Außendurchmesser der Hohlzylinder bzw. Ringtabletten von 3 bis 20 mm, bevorzugt 3 bis 10 mm, besonders bevorzugt 3 bis 7 mm, insbesondere 3,5 bis 6,5 mm, mit einem Innendurchmesser, der das 0,1- bis 0,7-fache des Außendurchmessers, und einer Länge, die das 0,2- bis 2-fache, bevorzugt das 0,3 bis 1,8-fache, besonders bevorzugt das 0,4 bis 1,6-fache des Außendurchmessers, beträgt und der Radius der Krümmung der Stirnflächen das 0,01 bis 0,5-fache, bevorzugt das 0,05 bis 0,4-fache, besonders bevorzugt das 0,1 bis 0,2-fache des Außendurchmessers beträgt.

Die Zeichnung 1 veranschaulicht einen möglichen Querschnitt der erfindungsgemäßen geformten Katalysatoren.

Die neue Katalysatorform ist auch vorteilhaft bei sogenannten Trägerkatalysatoren, die in der Weise hergestellt werden, daß die aktiven Komponenten durch Tränken, Eindampfen, Aufsprühen einer Lösung oder Suspension der Komponenten in der oxidischen oder Salzform auf den vorgeformten inerten Träger aufgebracht werden.

Hinsichtlich der chemischen Zusammensetzung der Katalysatoren bestehen keine Einschränkungen.

Die erfindungsgemäßen geformten Katalysatoren eignen sich für heterogen katalysierte Reaktionen, in denen sie z.B. fest angeordnet, als Schüttungen, bei Gasphasen- oder Flüssigphasenreaktionen, insbesondere bei stark exothermen Gasphasenreaktionen wie beispielsweise der Oxichlorierung von Ethylen zu 1,2-Dichlorethan, eingesetzt werden.

Die erfindungsgemäßen geformten Katalysatoren sind bevorzugt für die Oxichlorierung, insbesondere von Ethylen zu 1,2-Dichlorethan geeignet. Die Zusammensetzung von für die Oxichlorierung geeigneten Katalysatoren basiert auf einem Träger, der vorzugsweise aus einem hochoberflächigen Aluminiumoxid wie beispielsweise γ-oder δ-Al₂O₃ besteht. Die BET-Oberfläche liegt in der Regel zwischen 100 und 250 m²/g, vorzugsweise zwischen 150 und 220 m²/g. Vorzugsweise wird in einem ersten Schritt der Träger durch Tablettierung entsprechend der gewünschten Katalysatorgeometrie geformt, in einem folgenden Schritt werden Aktivkomponenten auf den geformten Träger aufgebracht. Im zur Tablettierung des Trägers verwendeten Ausgangsmaterial kann das hochoberflächige Aluminiumoxid auch ganz oder teilweise durch Verbindungen wie beispielsweise Pseudoböhmit ersetzt werden, die sich nach Calcinierung in ein hochoberflächiges Aluminiumoxid umwandeln. Daneben können auch Tablettierhilfsmittel wie beispielsweise Graphit oder Magnesiumstearat dem zu tablettierenden Ausgangsmaterial zugesetzt werden. Bevorzugt werden Tablettiermischungen wie in der WO-A 99/48606 beschrieben eingesetzt.

Die Tablettierung des Materials erfolgt erfindungsgemäß zu Hohlzylindern oder zu Ringtabletten. Die Hohlzylinder oder Ringe haben im arthmetischen Mittel in der Regel eine Höhe von 3 bis 7 mm, bevorzugt 4,8 bis 5,2 mm, besonders bevorzugt 4,9 bis 5,1 mm, einen Durchmesser von 3 bis 7 mm, bevorzugt 4,8 bis 5,2 mm, besonders bevorzugt 4,9 bis 5,1 mm und einen Durchmesser des Innenlochs von 2 bis 3,5 mm, bevorzugt 2,3 bis 2,7 mm, besonders bevorzugt 2,4 bis 2,6 mm.

Nach der Tablettierung werden die Katalysatorträger in der Regel calciniert. Die Calcinierung wird im allgemeinen bei 500 bis 800°C durchgeführt, vorzugsweise bei 700 bis 750°C. Die Calcinierung wird in der Regel in oxidierender Atmosphäre, im allgemeinen an Luft vorgenommen.

Durch das beschriebene Tablettierungsverfahren und die Auswahl der Einsatzstoffe wird in der Regel ein Katalysatorträger erhalten, der einerseits eine gute mechanische Stabilität und andererseits ein für die Oxichlorierung vorteilhaftes Porenvolumen aufweist. Die erfindungsgemäß erhaltenen Katalysatorträger für die Oxichlorierung haben in der Regel ein Porenvolumen von 0,1 bis 0,9 cm³/g, vorzugsweise von 0,3 bis 0,7 cm³/g, besonders bevorzugt von 0,4 bis 0,6 cm³/g und eine Seitendruckfestigkeit von mindestens 15 N, bevorzugt 22 bis 80 N, besonders bevorzugt 25 bis 60 N, insbesondere 27 bis 55 N.

Die so erhaltenen Träger können anschließend mit einer Lösung von CuCl₂ und gegebenenfalls weiteren Verbindungen wie beispielsweise KCl, anderen Salzen von Alkalimetallen, Eralkalimetallen, Seltenerdalkalimetallen getränkt werden. Gegebenenfalls kann auch Salzsäure zu einer solchen Lösung zugesetzt werden.

Das Volumen der Tränklösung wird vorteilhaft so gewählt, daß es 10 bis 200 % des Porenvolumens des Trägers entspricht, bevorzugt 30 bis 150 %, besonders bevorzugt 90 bis 110 %.

Nach der Tränkung werden die Katalysatorformlinge in der Regel bei 80 bis 300°C, vorzugsweise bei 100 bis 200°C getrocknet.

Die Konzentration und das Volumen der Tränklösung werden so gewählt, daß der Trägerkatalysator in der Regel einen Cu-Gehalt von 1 bis 15 Gew.-%, vorzugsweise von 2 bis 10 Gew.-% und ein K-Gehalt von 0,1 bis 8 Gew.-%, vorzugsweise von 0,3 bis 3 Gew.-% aufweist. Durch die Wahl der Metallkonzentrationen im Katalysator sowie gewünschtenfalls durch Verdünnung mit Inertmaterial wie Al₂O₃ kann das Aktivitätsprofil der Katalysatorpräparation wunschgemäß eingestellt werden. Der auf diese Weise erhältliche Trägerkatalysator eignet sich zur Herstellung von 1,2-Dichlorethan durch Oxichlorierung. Er verbindet einen geringen Druckverlust mit guter mechanischer Belastbarkeit.

Das Gesamtporenvolumen (Hg-Porosimetrie) der erfindungsgemäßen Katalysatorträger beträgt mindestens 0,3 ml/g, vorzugsweise mindestens 0,4 ml/g, und der Anteil von Makroporen daran beträgt mindestens 10%, bevorzugt 15 bis 50%, besonders bevorzugt 20 bis 40%.

Die erfindungsgemäßen geformten Katalysatoren eignen sich bevorzugt für die Oxichlorierung von Ethylen und in der Regel auch für andere heterogen katalysierte Reaktionen, beispielsweise für die Herstellung von Acrylsäure, Phthalsäureanhydrid und Maleinsäureanhydrid oder die Dehydrierung von Alkanen zu Alkenen, beispielsweise Propan zu Propen. Die Herstellung von erfindungsgemäß geformten Katalysatoren für solche und weitere Reaktionen kann abgesehen von der erfindungsgemäßen Formgebung analog zur Herstellung anderer für diese Reaktion geeigneter Katalysatoren erfolgen. Bei diesen Katalysatoren kann es sich um Träger- oder Vollkatalysatoren handeln.

Beispiel B1

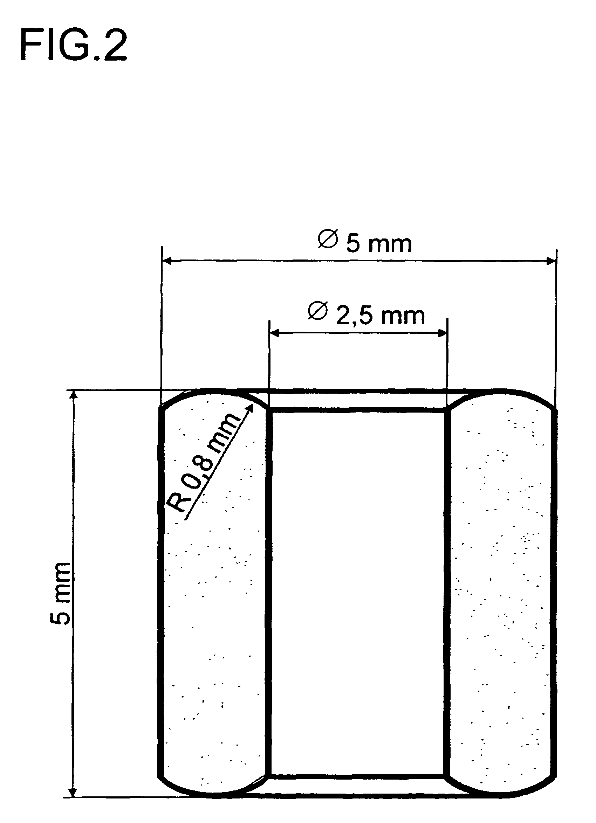

Eine Trockenmischung wie in der WO 99/48606 beschrieben wurde zu Ringtabletten mit den Abmessungen 5 mm x 5 mm x 2,5 mm (Höhe x Durchmesser x Durchmesser des Innenlochs) und gleichermaßen zum Außenrand und zum Rand der Innenbohrung hin abgerundeten Stirnflächen entsprechend Fig. 2 wurde auf eine Seitendruckfestigkeit von 20 N tablettiert und anschließend bei 700°C für zwei Stunden calciniert. Die physikalischen.Daten dieses Trägers sind in Tabelle 1 widergegeben.

### Beispiel B1*

Der Träger B1 wurde mit einer Lösung von Kupfer- und Kaliumchlorid auf einen Gehalt von 5,8 % Kupfer und 2,6 % Kalium getränkt. Dazu wurde jeweils eine entsprechend der Wasseraufnahme konzentrierte Lösung von Kupfer- und Kaliumchlorid auf die Träger gegeben und die getränkten Katalysatoren bei 120°C getrocknet.

Vergleichsbeispiel V1

Die gleiche Trockenmischung wie in B1 wurde in der WO 99/48606 beschrieben zu Ringtabletten mit den Abmessungen 5 mm x 5 mm x 2 mm (Höhe x Durchmesser x Durchmesser des Innenlochs) tablettiert und calciniert.

### Beispiel V1*

Der Träger V1 wurde mit einer Lösung von Kuper- und Kalumchlorid auf einen Gehalt von 5,8 % Kupfer und 2,6 % Kalium getränkt. Dazu wurde jeweils eine entsprechend der Wasseraufnahme konzentrierte Lösung von Kupfer- und Kaliumchlorid auf die Träger gegeben und die getränkten Katalysatoren bei 120°C getrocknet.

### Vergleichbeispiel V2

Die gleiche Trockenmischung wie in B1 wurde zu Ringtabletten mit den Abmessungen 5 mm x 5 mm x 2,5 mm (Höhe x Durchmesser x Durchmesser des Innenlochs) auf eine Seitendruckfestigkeit von 20 N tablettiert und anschließend bei 700°C für zwei Stunden calciniert.

### Vergleichbeispiel V3

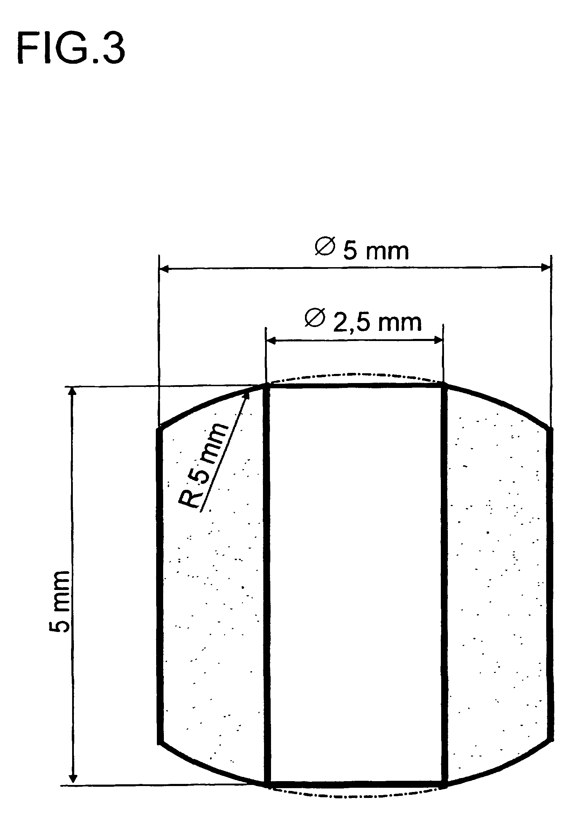

Die gleiche Trockenmischung wie in B1 wurde zu Ringtabletten mit den Abmessungen 5 mm x 5 mm x 2,5 mm (Höhe x Durchmesser x Durchmesser des Innenlochs) und abgerundeten Stirnflächen entsprechend Fig. 3 auf eine Seitendruckfestigkeit von 20 N tablettiert und calciniert.

### Druckverlustmessungen

Die Druckverlustmessungen wurden in einem Glasrohr mit 2,5 cm Durchmesser auf einen Meter Schüttlänge bestimmt. Ein Durchmesser in der Größenordnung von 2,5 cm ist typisch für die Reaktorrohre in technischen Rohrbündelreaktoren für stark exotherme heterogen katalysierte Gasphasenreaktionen wie beispielsweise die Oxichlorierung von Ethylen zu Ethylendichlorid. Um möglichst homogene Schüttungen und damit gut reproduzierbare Werte zu erhalten, wurden die Träger jeweils über einen Trichter so langsam zugegeben, dass die Träger nacheinander und praktisch einzeln aufeinander fielen.

### Performanceuntersuchungen

Die Katalysatoren Beipiel B1* und Vergleichbeispiel V1* wurden in einem Kreislaufreaktor bei einer Belastung von 1 Mol HCl / 90 ml Katalysator mit einem stöchiometrischen Feed von Chlorwasserstoff, Ethylen und Luft getestet. In Tabelle 3 sind der Ethylen-Umsatz und die Verbrennungsselektivität wiedergegeben.

**Tabelle 1: Physikalische Daten der Träger**

| | Seitendruckhärte [N] | Porenvolumen [ml/g] | BET-Oberfläche [m²/g] | Wasseraufnahme [ml/g] |
|---|---|---|---|---|
| Beispiel B1 | 25 | 0,42 | 160 | 0,48 |
| Vergleichsbeispiel V1 | 31 | 0,43 | 197 | 0,52 |
| Vergleichsbeispiel V2 | 24 | 0,41 | 177 | 0,50 |
| Vergleichsbeispiel V3 | 25 | 0,41 | 170 | 0,51 |

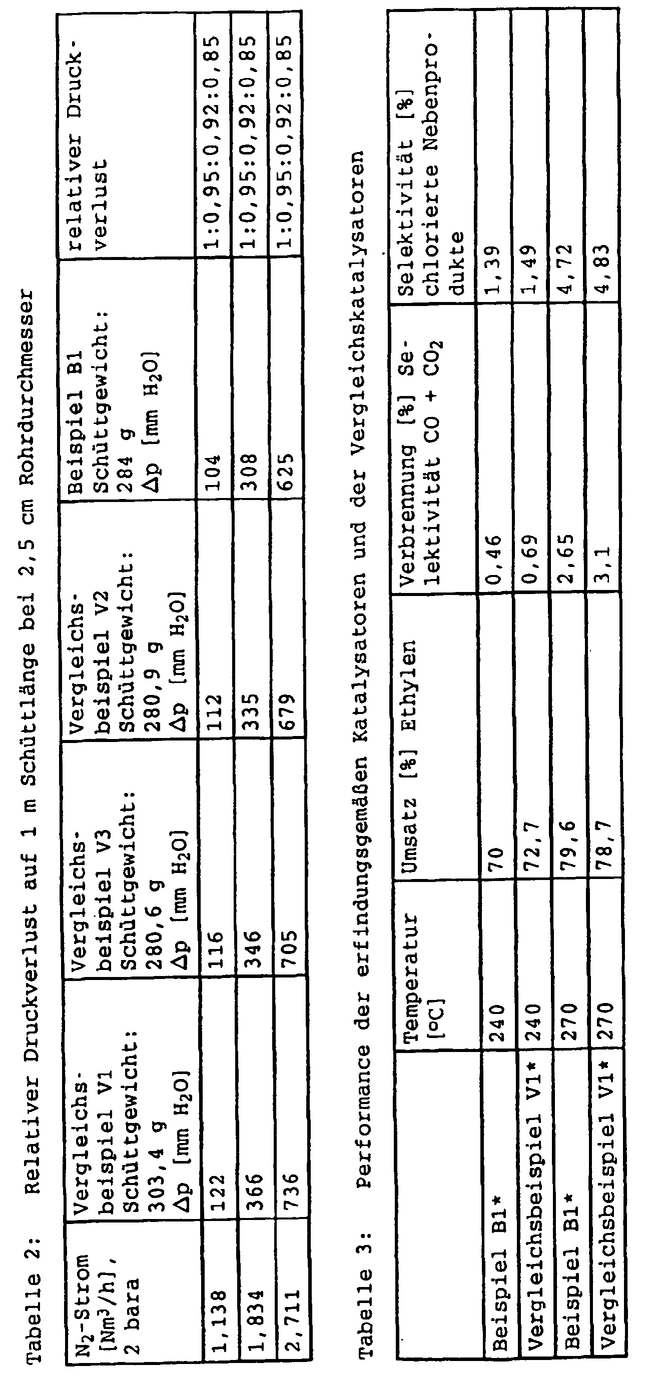

Der Druckverlust der erfindungsgemäßen geformten Katalysatoren liegt um ca. 15% unter dem in der WO-A-99/48606 beschriebenen Katalysator in Form von 5 mm x 5 mm x 2 mm-Ringtabletten (Vergleichsbeispiel VI). Auch gegenüber den Trägergeometrien der Vergleichbeispiele V2 und V3 zeichnet sich die erfindungsgemäße Form des Trägers aus Beispiel B1 durch einen deutlich verringerten Druckverlust 11 % bzw. 8 % aus. Der Träger V2 ist eine im Hinblick auf den Druckverlust für den Fachmann naheliegende sich aus der Beschreibung der WO-A 99/48606 ergebende Verbesserung des Trägers V1. V3 ist eine Veränderung des Trägers V2 gemäß der Lehre aus EP-A-184 790. Da alle verglichenen Träger nahezu gleiche Außenabmessungen aufweisen und bei gleichem und für technische Reaktorrohre typischem Rohrdurchmesser untersucht wurden, zeigen dieser Ergebnisse sehr deutlich den verbesserten Druckverlust von Katalysatoren der erfindungsgemäßen Formgebung.

Den Vorteil der erfindungsgemäßne Trägergeometrie für die Performance von entsprechender Katalysatoren bei der Oxichlorierung zeigt der Vergleich der Katalysatoren V1* und B1*. Der Katalysator mit erfindungsgemäßer Formgebung (B1*) zeigt bei annähernd gleichem Umsatz eine deutlich verbesserte Selektivität gegenüber dem Katalysator mit bekannter Formgebung (V1).

## Patentansprüche

1. Geformte Katalysatoren für heterogen katalysierte Reaktionen in Form von Hohlzylindern oder Ringtabletten, **dadurch gekennzeichnet, dass** im Querschnitt die äußere Wandfläche (Außenrand) und die innere Wandfläche (Rand der Innenbohrung) weitgehend eben und parallel zueinander angeordnet sind, während die Stirnflächen durchgehend so abgerundet sind, dass - entlang der Innenbohrung betrachtet - sich der Katalysatorkörper sowohl von Außenrand als auch vom Rand der Innenbohrung her verjüngt.

2. Geformte Katalysatoren für heterogen katalysierte Reaktionen nach Anspruch 1, hergestellt durch Tränkung eines geformten γ-Al₂O₃-Trägers mit einer BET-Oberfläche von 100 bis 250 g/m² mit Salzen von Kupfer und gegebenenfalls weiteren Elementen.

3. Geformte Katalysatoren für heterogen katalysierte Reaktionen nach Anspruch 2, **dadurch gekennzeichnet, daß** die durch Tränkung aufgebrachten Elemente in Form ihrer Chloride vorliegen.

4. Verfahren zur Herstellung von geformten Katalysatoren für heterogen katalysierte Reaktionen nach Anspruch 1, **dadurch gekennzeichnet, daß** man geformte γ-Al₂O₃-Träger mit einer BET-Oberfläche von 100 bis 250 g/m² mit Salzlösungen von Kupfer, Kalium und gegebenenfalls weiteren Elementen behandelt.

5. Verwendung der geformten Katalysatoren nach einem der Ansprüche 1, 2 oder 3 für exotherme Gasphasenreaktionen.

6. Verwendung der geformten Katalysatoren nach einem der Ansprüche 1, 2 oder 3 für Oxichlorierungsreaktionen.

7. Verwendung der geformten Katalysatoren nach einem der Ansprüche 1, 2 oder 3 für die Oxichlorierung von Ethylen zu 1,2-Dichlorethan.

8. Verfahren zur Herstellung von 1,2-Dichlorethan, **dadurch gekennzeichnet, daß** man geformte Katalysatoren gemäß einem der Ansprüche 1, 2 oder 3 einsetzt.

9. Verwendung der geformten Katalysatoren nach einem der Ansprüche 1, 2 oder 3 für partielle Oxidationsreaktionen.

## Claims

1. A shaped catalyst for heterogeneously catalyzed reactions in the form of hollow cylinders or annular tablets, wherein, in cross section, the outer wall (outer surface) and the internal wall (surface of the central hole) are largely flat and parallel to one another while the end faces are rounded throughout so that, viewed along the central hole, the catalyst body tapers both from the outer surface and from the surface of the central hole.

2. The shaped catalyst for heterogeneously catalyzed reactions according to claim 1, produced by impregnating a shaped γ-Al₂O₃ support having a BET surface area of from 100 to 250 g/m² with salts of copper and, if appropriate, further elements.

3. The shaped catalyst for heterogeneously catalyzed reactions according to claim 2, wherein the elements applied by impregnation are present in the form of their chlorides.

4. A process for producing a shaped catalyst for heterogeneously catalyzed reactions according to claim 1, which comprises treating shaped γ-Al₂O₃ supports having a BET surface area of from 100 to 250 g/m² with salt solutions of copper, potassium and, if appropriate, further elements.

5. The use of a shaped catalyst according to any of claims 1, 2 or 3 for exothermic gas-phase reactions.

6. The use of a shaped catalyst according to any of claims 1, 2 or 3 for oxychlorination reactions.

7. The use of a shaped catalyst according to any of claims 1, 2 or 3 for the oxychlorination of ethylene to give 1,2-dichloroethane.

8. A process for preparing 1,2-dichloroethane, wherein a shaped catalyst according to any of claims 1, 2 or 3 is used.

9. The use of a shaped catalyst according to any of claims 1, 2 or 3 for partial oxidation reactions.

## Revendications

1. Catalyseurs mis en forme pour des réactions catalysées de manière hétérogène sous forme de cylindres creux ou de tablettes annulaires, **caractérisés en ce que**, en coupe, la face extérieure de paroi (bord extérieur) et la face intérieure de paroi (bord du trou intérieur) sont agencées l'une par rapport à l'autre plates et parallèles, tandis que les faces avant sont entièrement arrondies, de sorte que, vu le long du trou intérieur, le corps de catalyseur s'amincit du bord extérieur comme du bord du trou intérieur.

2. Catalyseurs mis en forme pour des réactions catalysées de manière hétérogène selon la revendication 1, fabriqués par imprégnation d'un support de γ-Al₂O₃ mis en forme présentant une surface BET de 100 à 250 g/m² avec des sels de cuivre et le cas échéant d'autres éléments.

3. Catalyseurs mis en forme pour des réactions catalysées de manière hétérogène selon la revendication 2, **caractérisés en ce qu'**ils sont présents sous forme de leurs chlorures par l'imprégnation des éléments appliqués.

4. Procédé de fabrication de catalyseurs mis en forme pour des réactions catalysées de manière hétérogène selon la revendication 1, **caractérisé en ce que** l'on traite des supports de γ-Al₂O₃ mis en forme présentant une surface BET de 100 à 250 g/m² avec des solutions salines de cuivre, de potassium et le cas échéant d'autres éléments.

5. Utilisation des catalyseurs mis en forme selon l'une quelconque des revendications 1, 2 ou 3 pour des réactions en phase gazeuse exothermiques.

6. Utilisation des catalyseurs mis en forme selon l'une quelconque des revendications 1, 2 ou 3 pour des réactions d'oxychloration.

7. Utilisation des catalyseurs mis en forme selon l'une quelconque des revendications 1, 2 ou 3 pour l'oxychloration d'éthylène en 1,2-dichloroéthane.

8. Procédé de fabrication de 1,2-dichloroéthane, **caractérisé en ce qu'**on met en oeuvre des catalyseurs mis en forme selon l'une quelconque des revendications 1, 2 ou 3.

9. Utilisation des catalyseurs mis en forme selon l'une quelconque des revendications 1, 2 ou 3 pour des réactions d'oxydation partielle.
